# EUROPEAN PATENT APPLICATION

(11) **EP 4 151 712 A1**
(43) Date of publication of application: **22.03.2023**
(21) Application number: 22775813.3
(22) Date of filing: 25.03.2022
(51) Int. Cl.: C12M 1/00, G01N 33/53, G01N 33/547, G01N 21/63

(54) **SUBSTRATE FOR IMMOBILIZING SUBSTANCE, AND USE THEREOF**

(30) Priority: 26.03.2021 JP 2021054300
(71) Applicant: RIKEN, Wako-shi, Saitama 351-0198 (JP); R-Nanobio Co. Ltd., Wako-shi, Saitama 351-0104 (JP)
(72) Inventor: ITO, Yoshihiro, Wako-shi, Saitama 351-0104 (JP)
(74) Representative: Plasseraud IP
(86) International application number: PCT/JP2022/014255
(87) International publication number: WO 2022/203032

(57) **Abstract**

A substrate in accordance with an embodiment of the present invention is a substrate that has a surface to which a substance is to be immobilized and on which a layer of a substance immobilizing agent is formed. The substance immobilizing agent has photoreactive groups and contains a water-soluble polymer or a water-soluble monomer which turns into the water-soluble polymer by polymerization, and a thickness of the layer of the substance immobilizing agent is not less than 3 nm and less than 500 nm.

## Description

### Technical Field

The present invention relates to a substrate for immobilizing thereto a desired substance such as a polypeptide, a protein, an enzyme, a nucleic acid, or a lipid, and also relates to use of the substrate.

### Background Art

Immunoplates for immunoassays, in which immunoplates antibodies or antigens are immobilized to plates, DNA chips, in which nucleic acids are immobilized to chips, and the like have been widely used.

As a method for immobilizing a protein or a nucleic acid to a substrate, various methods have been developed. For example, Patent Literature 1 discloses coating a substrate with an aqueous solution which contains a substance immobilizing agent and a substance to be immobilized to the substrate and then photo-irradiating the substrate so as to immobilize the substance to the substrate. As the substance immobilizing agent, a substance immobilizing agent which contains a water-soluble polymer having a photoreactive group such as an azide group is specifically disclosed.

### Citation List

### [Patent Literature]

[Patent Literature 1]
Pamphlet of International Publication No. WO 2004/088319

### Summary of Invention

### Technical Problem

With regard to a substance immobilizing agent which has a photoreactive group and contains a water-soluble polymer, the photoreactive group fulfills a role of immobilizing a substance to a substrate. The water-soluble polymer is presumed to fulfill a role of, for example, preventing non-specific adsorption to the substrate. However, in a case where a substrate to which a substance is immobilized by such a substance immobilizing agent is used to test an interaction between the substance and a sample, there are still a lot of uncertainty about conditions under which a favorable signal is obtained. Thus, there is room for further improvement.

The present invention has been made in view of the above problem, and an object thereof is to provide (i) a substrate for immobilizing a substance thereto, which substrate has improved characteristics in connection with detection of an interaction with a substance immobilized thereto and a sample, and (ii) use of the substrate.

### Solution to Problem

In order to attain the above object, the present invention includes the following aspect:
1) A substrate that has a surface to which a substance is to be immobilized and on which a layer of a substance immobilizing agent is formed, wherein the substance immobilizing agent has photoreactive groups and contains a water-soluble polymer or a water-soluble monomer which turns into the water-soluble polymer by polymerization, and a thickness of the layer of the substance immobilizing agent is not less than 3 nm and less than 500 nm.

### Advantageous Effects of Invention

According to an aspect of the present invention, it is possible to provide (i) a substrate for immobilizing a substance thereto, which substrate is obtained with use of a substance immobilizing agent that has photoreactive groups and contains a water-soluble polymer or a water-soluble monomer and which substrate has improved characteristics and (ii) use of the substrate.

### Description of Embodiments

Substance immobilizing agents in accordance with embodiments of the present invention share the following features: 1) each of the substance immobilizing agents has photoreactive groups; and 2) each of the substance immobilizing agents contains a water-soluble polymer or a water-soluble monomer which turns into the water-soluble polymer by polymerization.

Each of the substance immobilizing agents in accordance with embodiments of the present invention is water-soluble. The solubility of each of the substance immobilizing agents in water or a mixed solvent containing 90 mass% of water (grams of each of the substance immobilizing agents which can be dissolved in 100 g of water) is preferably not less than 5.

The molecular weight of the water-soluble polymer contained in each of the substance immobilizing agents is preferably 5×10² to 5×10⁶, more preferably 7×10² to 1×10⁶, and still more preferably 1×10³ to 5×10⁵.

The water-soluble polymer is used, for example, as a substance immobilizing agent in a state of being dissolved in a hydrophilic solvent. Examples of the hydrophilic solvent include water, methanol, ethanol, acetone, and mixed solvents of any of these solvents. The concentration of the water-soluble polymer is not limited in particular, and is, for example, approximately 0.005% to 10%, and preferably approximately 0.05% to 5%.

Each of the substance immobilizing agents in accordance with embodiments of the present invention enables a plurality of kinds of substances to be immobilized. For example, it is possible to immobilize a plurality of kinds of nucleic acids, proteins, and biological extracts, and also possible to immobilize polyclonal antibodies and/or monoclonal antibodies.

The following description will discuss the substance immobilizing agents in detail, while taking substance immobilizing agents (A), (B), (C), and (D) as examples.

### [1-1. Example of substance immobilizing agent (A)]

In the substance immobilizing agent (A) in accordance with an embodiment of the present invention, the water-soluble polymer is constituted by a polymer which has, in one molecule thereof, at least two photoreactive groups and of which the molecule is electrically neutral or the water-soluble polymer has a main chain which contains a polyoxyethylene structure or a polyoxyalkylene structure.

Note that the expression "entire molecule is electrically neutral" means that the molecule does not have a group which is ionized in an aqueous solution having a pH in the vicinity of neutral (pH 6 to 8) to become an ion or that, even in a case where the molecule has such groups, the molecule has both a group which becomes a cation and a group which becomes an anion such that the total of the electric charges of these groups is substantially 0 (zero). Note, here, that the term "substantially" means that the total of the electric charges is 0 (zero) or that, even in a case where the total is not 0 (zero), the total is so small that the electric charges do not adversely affect the effect of the present invention.

### (Water-soluble polymer)

The number of the photoreactive groups per molecule of the water-soluble polymer is not particularly limited, provided that the number is two or more. The photoreactive groups are contained in an amount of preferably not less than 2.5 mol%, more preferably not less than 5 mol%, still more preferably not less than 10 mol%, even more preferably not less than 15 mol%, and particularly preferably not less than 20 mol%, with respect to the entire water-soluble polymer. From the viewpoint of, for example, easiness of dissolution of the water-soluble polymer in a volatile polar solvent, the photoreactive groups are contained in an amount of preferably less than 50 mol%, more preferably not more than 45 mol%, still more preferably not more than 40 mol%, and even more preferably not more than 30 mol%, with respect to the entire water-soluble polymer. As used herein, the expression "the photoreactive groups contained in an amount (mol%) ... with respect to the entire water-soluble polymer" indicates the proportion, represented by a percentage, of the number of repeating units each having a photoreactive group to the total number of repeating units constituting the water-soluble polymer. The photoreactive groups may be each directly bonded to the main chain of the water-soluble polymer. Alternatively, the photoreactive groups may be each bonded to the main chain of the water-soluble polymer with any spacer structure therebetween. Usually, the latter is preferable from the viewpoint of, for example, easiness of production.

The spacer structure is not limited in any way, and examples thereof can include C1-10 alkylene groups (in which substitution with one or two hydroxy groups may be present) and phenylene groups (in which substitution with one to three C1-4 alkyl groups or one to three hydroxy groups may be present).

Introduction of the photoreactive groups into the main chain of the water-soluble polymer can be easily carried out in accordance with a conventional method. Alternatively, in a case where the water-soluble polymer is a water-soluble polymer which is formed by polymerization of a monomer as with the case of a water-soluble vinyl-based polymer, it is also possible to produce a nonionic water-soluble polymer which has the photoreactive groups, by subjecting, to copolymerization, a photoreactive vinyl-based monomer and a vinyl-based monomer which turns into a main constitutional unit of a water-soluble vinyl-based polymer. Preferable examples of a photoreactive water-soluble vinyl-based polymer obtained by this method can include poly((meth)acrylamide-photoreactive (meth)acrylic acid amide) copolymers, poly(glycidyl (meth)acrylate-photoreactive (meth)acrylic acid amide) copolymers, and poly(ethylene glycol mono(meth)acrylate-photoreactive acrylic acid amide) copolymers. With regard to the substance immobilizing agent (A), the pamphlet of International Publication No. WO 2004/088319 (Japanese Patent No. 4630817) also describes some examples thereof, and this description can be referred to as appropriate.

Examples of a polymerizable group can include groups each having a double bond(s) or a triple bond(s) (e.g., (meth)acrylate groups, vinyl groups, acrylic groups, acryloyl groups, methacryloxy groups, methacrylamide groups, acryloxy groups, acrylamide groups, styryloxy groups, and styrylamide groups). The water-soluble monomer may contain, for example, one to three, preferably one or two polymerizable groups in one molecule thereof.

### (Main chain of water-soluble polymer)

Since the main chain of the water-soluble polymer contains the polyoxyethylene structure (structure in which -C-C-O- is repeated), the main chain of the water-soluble polymer is hydrophilic. By having a hydrophilic main chain, the water-soluble polymer maintains its water-solubility even in a case where the water-soluble polymer has more hydrophobic photoreactive groups than a conventional water-soluble polymer. Therefore, by dissolving this water-soluble polymer in water or a hydrophilic solvent (e.g., a lower alcohol such as methanol or ethanol) and using a resultant solution as a substance immobilizing agent, it is possible to relatively increase the amount (concentration) of the photoreactive groups that can be contained in the substance immobilizing agent. By causing the water-soluble polymer to contain more photoreactive groups, it is possible to increase the number of sites (crosslinking points) at each of which a covalent bond with a substance to be immobilized can be formed. Therefore, it is possible to more firmly immobilize, to a substrate, a substance to be immobilized, and possible to increase the sensitivity of detection of the substance.

The main chain of the water-soluble polymer preferably contains, as repeating units, a structure represented by formula (1) and a structure represented by formula (2). Alternatively, the main chain of the water-soluble polymer may have, as repeating units, a structure represented by formula (3) and a structure represented by formula (4).

-(R¹-R¹²)- ... (1)

-(R²-R¹²)- ... (2)

-(R¹-R¹²-O)- ... (3)

-(R²-R¹²-O)- ... (4)

In each of formulae (1) and (3), R¹ is a group containing a photoreactive group.

In each of formulae (2) and (4), R² is a zwitterionic group, an amide group, an imidazole group, a polyoxyalkylene group, a water-soluble functional group, a group which has a chemical structure containing a combination of any of these groups, or a hydrogen atom.

Hydrogen atoms bonded to carbon atoms constituting the main chain represented by formulae (1), (2), (3), and (4) may be each independently substituted with a substituent selected from the group consisting of halogen atoms (e.g., fluorine atom, chlorine atom, bromine atom, and iodine atom), C1-4 alkyl groups, and C1-4 haloalkyl groups (examples of halogen atoms are the same as those listed above).

Note that halogen atoms and C1-4 haloalkyl groups in the following description have the same definitions unless otherwise specified.

### (R¹)

R¹ is a group containing a photoreactive group. Examples of the photoreactive group include azide groups, acetyl groups, benzoyl groups, diazo groups, diazirine groups, ketone groups, and quinone groups. R¹ is preferably a group containing a photoreactive group selected from acetyl groups, benzoyl groups, azide groups (groups each represented by -N₃), and diazirynyl groups (diazirine group: a group which has a three-membered ring structure formed by one carbon atom being bonded to two nitrogen atoms that are double-bonded and in which the carbon atom has bonding arms). R¹ is more preferably a group containing a photoreactive group selected from azide groups and diazirynyl groups. R¹ is still more preferably a group containing a photoreactive group selected from aromatic azide groups and aromatic diazirynyl groups. Examples of the aromatic azide groups include substituted or unsubstituted phenyl azide groups, and examples of the aromatic diazirynyl groups include substituted or unsubstituted phenyl diazirynyl groups. As described above, a hydrogen atom in each of formulae (1) and (3) (which hydrogen atom may be a hydrogen atom in R¹) may be substituted with a substituent. Examples of the substituent include halogen atoms, C1-C4 alkyl groups, and C1-4 haloalkyl groups.

In an aspect, R¹ is -R³R⁴, where R³ is a linker containing a carbon atom in the main chain thereof and R⁴ is an azide group or a diazirynyl group. In another aspect, R³ is a linker containing a carbon atom and a heteroatom in the main chain thereof. The heteroatom contained in R³ can contribute to, for example, 1) an improvement in hydrophilicity and 2) suppression of non-specific adsorption of a biological sample or the like. The number of atoms constituting the main chain of the linker is not particularly limited, but is, for example, 1 to 20, preferably 1 to 10, and, for example, 1 to 6. Examples of the heteroatom include an oxygen atom, a nitrogen atom, a sulfur atom, a silicon atom, and a phosphorus atom. The heteroatom is preferably selected from an oxygen atom and a nitrogen atom. In a case where the linker contains the heteroatom in the main chain thereof, the number of heteroatoms is not limited in particular, but is, for example, one to three, or one or two.

More specific examples of R³ are a linker having an ether group (-(CH₂)n₁-O-(CH₂)n₂-), a linker having an amide group (-NH-C(O)-), a linker having an alkylene group, and a linker having a chemical structure containing a combination of any of these groups. Examples of R³ include: *-NH-C(O)-; *-C(O)-NH-(CH₂)n-NH-C(O)-; and *-(CH₂)n₁-O-(CH₂)n₂-. Note that * indicates a bond with R⁴. Note, here, that n, n₁, and n₂ are each independently an integer of, for example, 1 to 5, 1 to 3, or 1 or 2. Hydrogen atoms may be each independently substituted with a substituent selected from the group consisting of halogen atoms, C1-4 alkyl groups, and C1-4 haloalkyl groups.

Specific examples of R¹ include groups shown below. Note that a group obtained by substituting a fluorine atom in any of the groups below with another halogen atom, a group obtained by substituting at least one or some (one to four) of hydrogen atoms on a benzene ring in any of the groups below with a halogen atom(s), and the like are also preferable examples.

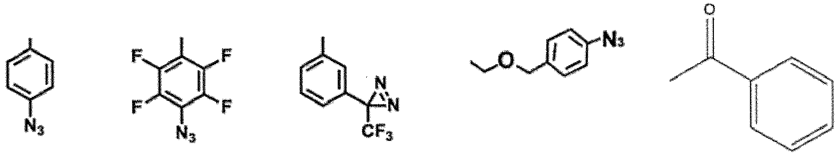

### (R2)

R² is a zwitterionic group, an amide group (-NH-C(O)-), a polyoxyalkylene group (preferably a polyoxyethylene group), a water-soluble functional group, a group which has a chemical structure containing a combination of any of these groups, or a hydrogen atom. A hydrogen atom in each of formulae (2) and (4) (which hydrogen atom may be a hydrogen atom in R²) may be substituted with a substituent. Examples of the substituent include halogen atoms, C1-C4 alkyl groups, and C1-4 haloalkyl groups. In a case where the above amide group constitutes a part of a lactam ring and a nitrogen atom in the lactam ring is bonded to a carbon atom in each of formulae (2) and (4), no hydrogen atom is present in the amide group. These groups can contribute to 1) an improvement in hydrophilicity and 2) suppression of non-specific adsorption of a biological sample or the like. Examples of the water-soluble functional group include sulfonic acid groups, carboxyl groups, and hydroxy groups.

R² (non-specific interaction suppressing group) may be directly bonded to the main chain of the water-soluble polymer. Alternatively, R² may be bonded to the main chain of the water-soluble polymer with any spacer structure therebetween. Usually, the latter is preferable from the viewpoint of, for example, easiness of production.

### (R¹²)

R¹² may be a single bond, a C1-10 alkylene group (in which substitution with one or two hydroxy groups may be present), or a phenylene group (in which substitution with one to three C1-4 alkyl groups or one to three hydroxy groups may be present).

In terms of suppression of non-specific adsorption of a biological sample or the like, the zwitterionic group preferably has a sulfobetaine structure, a phosphobetaine structure, or an imidazole betaine structure.

The sulfobetaine structure typically indicates a structure which has -SO₃- and a positively charged atom and in which - SO₃- and the positively charged atom are not in adjacent positions in the zwitterionic group (i.e., present with another atom therebetween) and no dissociable hydrogen atom is bonded to the positively charged atom. The positively charged atom is, for example, a nitrogen atom having a quaternary ammonium structure. An example of the sulfobetaine structure is -(CH₂)ₙ-N⁺(R)₂-(CH₂)ₙ-SO₃⁻, where each R is independently a C1-C4 alkyl group and each n is independently an integer of 1 to 5 (preferably 1 to 3).

The phosphobetaine structure typically indicates a structure which has a phosphodiester bond (one oxygen atom is negatively charged) and a positively charged atom and in which the phosphodiester bond and the positively charged atom are not in adjacent positions in the zwitterionic group (i.e., present with another atom therebetween) and no dissociable hydrogen atom is bonded to the positively charged atom. The positively charged atom is, for example, a nitrogen atom having a quaternary ammonium structure. An example of the phosphobetaine structure is -R¹¹-PO₄⁻-(CH₂)ₙ-N⁺(R)₃, where each R is independently a C1-C4 alkyl group, and n is an integer of 1 to 5 (preferably 1 to 3). -PO₄⁻- indicates the phosphodiester bond. In the formula, R¹¹ is (CH₂)ₙ₁, a single bond, or a C1-10 alkylene group (in which substitution with one or two hydroxy groups may be present), and n1 is an integer of 1 to 5 (preferably 1 to 3).

Examples of the phosphobetaine structure can include units each derived from (i.e., obtained by polymerizing any of the following units) 2-methacryloyloxyethyl phosphorylcholine, 2-acryloyloxyethyl phosphorylcholine, N-(2-methacrylamide)ethyl phosphorylcholine, 4-methacryloyloxybutyl phosphorylcholine, 6-methacryloyloxyhexyl phosphorylcholine, 10-methacryloyloxydecyl phosphorylcholine, ω-methacryloyldioxyethylene phosphorylcholine or 4-styryloxybutyl phosphorylcholine. Of these units, a unit derived from 2-methacryloyloxyethyl phosphorylcholine is particularly preferable.

The imidazole betaine structure typically indicates a structure which has a negatively charged atom and an imidazole ring that has one positively charged nitrogen atom and in which the negatively charged atom and the imidazole ring are not in adjacent positions in the zwitterionic group (i.e., present with another atom therebetween) and no dissociable hydrogen atom is bonded to the positively charged nitrogen atom. The negatively charged atom is, for example, an oxygen atom having the structure of a carboxyl group. An example of the imidazole betaine structure is -(CH₂)ₙ-HC(COO-)-(CH₂)ₙ-(IM), where (CH₂)ₙ is (CH₂)ₙ but one of n (CH₂) may be (NH), and each n is independently an integer of 1 to 5 (preferably 1 to 3). In the formula, (IM) is the imidazole ring that has one positively charged nitrogen atom, and a carbon atom on the imidazole ring is bonded to (CH₂)ₙ-.

The sulfobetaine structure, the phosphobetaine structure, or the imidazole betaine structure contains a phosphorylcholine group. A phosphorylcholine-containing polymer was invented based on the conception that non-specific adsorption may be effectively prevented by immobilizing a desired substance to a substrate with use of a polymer containing phosphorylcholine, which is a constituent of biomembrane, focusing attention on the fact that biomembranes hardly accompany non-specific adsorption in spite of coming into contact with various substances.

R² may contain a heterocyclic ring structure containing nitrogen. Examples of the heterocyclic ring structure containing nitrogen include imidazole rings, pyrrolidone rings, pyrrole rings, pyridine rings, pyrimidine rings, and, lactam rings (y-lactam ring, δ-lactam ring, and the like).

R² preferably contains a structure represented by formula (5) or (6).

-C(O)-R⁵ ... (5)

-C(O)-OR⁵ ... (6)

In each of formulae (5) and (6), R⁵ is a zwitterionic group, an imidazole group, a polyoxyalkylene group, or a water-soluble functional group as described above. The polyoxyalkylene group is particularly preferably a polyoxyethylene group (-(C₂H₄O)ₙ-H). n is, for example, an integer of 1 or more, and preferably 10 to 1000, and the molecular weight of (C₂H₄O)ₙ is several hundreds to several tens of thousands.

Specific examples of R² include groups shown below. Note that n in the formula is, for example, 1 or more, and preferably 10 to 1000, and the molecular weight of (C₂H₄O)ₙ is, several hundreds to several tens of thousands.

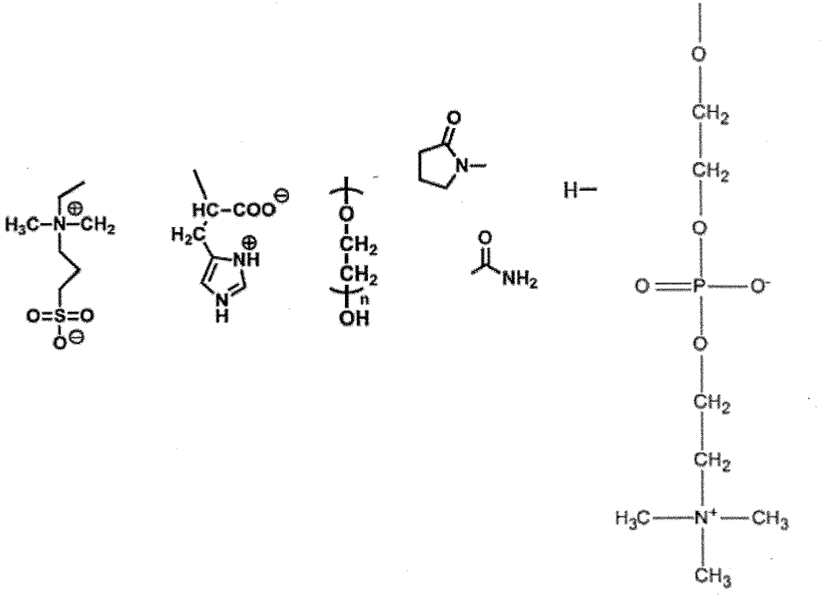

(Examples of preferable combination of structure represented by formula (3) and structure represented by formula (4))
- Combination example <1>: In formula (3), R¹ is -R³R⁴, where R³ is a linker containing a carbon atom in the main chain thereof and R⁴ is an azide group or a diazirynyl group, and in formula (4), R² is a zwitterionic group, an amide group, an imidazole group, a polyoxyalkylene group, a water-soluble functional group, a group which has a chemical structure containing a combination of any of these groups, or a hydrogen atom.
- Combination example <2>: In formula (3), R¹ is -R³R⁴, where R³ is a linker containing a carbon atom in the main chain thereof and R⁴ is an azide group or a diazirynyl group, and in formula (4), R² contains the structure represented by formula (5) or (6) and R⁵ is a zwitterionic group, an amide group, an imidazole group, a polyoxyalkylene group, a water-soluble functional group, or a group which has a chemical structure containing a combination of any of these groups.
- Combination example <3>: In formula (3), R¹ is -R³R⁴, where R³ is a linker containing a carbon atom and a heteroatom in the main chain thereof, and in formula (4), R² is a zwitterionic group, an amide group, an imidazole group, a polyoxyalkylene group, a water-soluble functional group, a group which has a chemical structure containing a combination of any of these groups, or a hydrogen atom.
- Combination example <4>: In formula (3), R¹ is -R³R⁴, where R³ is a linker containing a carbon atom and a heteroatom in the main chain thereof, and in formula (4), R² contains the structure represented by formula (5) or (6) and R⁵ is a zwitterionic group, an imidazole group, an amide group, a polyoxyalkylene group, a water-soluble functional group, or a group which has a chemical structure containing a combination of any of these groups.
- Combination example <5>: In formula (3), R¹ is -R³R⁴, where R³ is a linker having an ether group, a linker having an amide group, a linker having an alkylene group, or a linker having a chemical structure containing a combination of any of these groups, and in formula (4), R² is a zwitterionic group, an amide group, an imidazole group, a polyoxyalkylene group, a water-soluble functional group, a group which has a chemical structure containing a combination of any of these groups, or a hydrogen atom.
- Combination example <6>: In formula (3), R¹ is -R³R⁴, where R³ is a linker having an ether group, a linker having an amide group, a linker having an alkylene group, or a linker having a chemical structure containing a combination of any of these groups, and in formula (4), R² contains the structure represented by formula (5) or (6) and R⁵ is a zwitterionic group, an amide group, an imidazole group, a polyoxyalkylene group, a water-soluble functional group, or a group which has a chemical structure containing a combination of any of these groups.

A preferable example of the water-soluble polymer used in an embodiment of the present invention is a nonionic water-soluble polymer having, in one molecule thereof, at least two photoreactive groups.

Note, here, that the term "nonionic" means that the polymer substantially does not have a group which is ionized in an aqueous solution having a pH in the vicinity of neutral (pH 6 to 8) to become an ion. Note also that the term "substantially" means that the polymer does not have such a group at all or that, even in a case where the polymer has such a group, the amount of the group is so small (for example, the number of such groups is not more than 1% of the number of carbon atoms) that the group does not adversely affect the effect of the present invention.

Preferable examples of such a nonionic water-soluble polymer can include polyalkylene glycols such as polyethylene glycol (PEG) and polypropylene glycol; and nonionic vinyl-based polymers each containing, as a constituent, one or a combination of any of the following monomers: vinyl alcohol, methyl vinyl ether, vinyl pyrrolidone, vinyl oxazolidone, vinyl methyl oxazolidone, N-vinylsuccinimide, N-vinylformamide, N-vinyl-N-methylformamide, N-vinylacetamide, N-vinyl-N-methylacetamide, 2-hydroxyethyl methacrylate, acrylamide, methacrylamide, N,N-dimethylacrylamide, N-isopropylacrylamide, diacetoneacrylamide, methylolacrylamide, acryloylmorpholine, acryloylpyrrolidine, acryloylpiperidine, styrene, chloromethylstyrene, bromomethylstyrene, vinyl acetate, methyl methacrylate, butyl acrylate, methyl cyanoacrylate, ethyl cyanoacrylate, n-propyl cyanoacrylate, iso-propyl cyanoacrylate, n-butyl cyanoacrylate, iso-butyl cyanoacrylate, tert-butyl cyanoacrylate, glycidyl methacrylate, ethyl vinyl ether, n-propyl vinyl ether, iso-propyl vinyl ether, n-butyl vinyl ether, iso-butyl vinyl ether, and tert-butyl vinyl ether. Note, however, that the nonionic water-soluble polymer is not limited to these nonionic water-soluble polymers.

Of these nonionic water-soluble polymers, polyethylene glycol, poly(meth)acrylamide, and poly(glycidyl (meth)acrylate) are particularly preferable. Of these nonionic water-soluble polymers, polyethylene glycol is particularly preferable. Furthermore, a vinyl polymer of polyethylene glycol (meth)acrylate, which has a hydrophobic main chain and a hydrophilic side chain that is attached to the hydrophobic main chain, has the most preferable effect. As used herein, the term "(meth)acryl" means "methacryl" or "acryl", and the term "(meth)acrylate" means acrylate or methacrylate.

Preferable examples of the water-soluble polymer include water-soluble polymers containing a structure represented by the following formula (7) and water-soluble polymers containing a structure represented by the following formula (8).

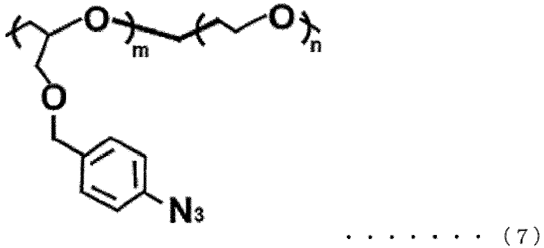

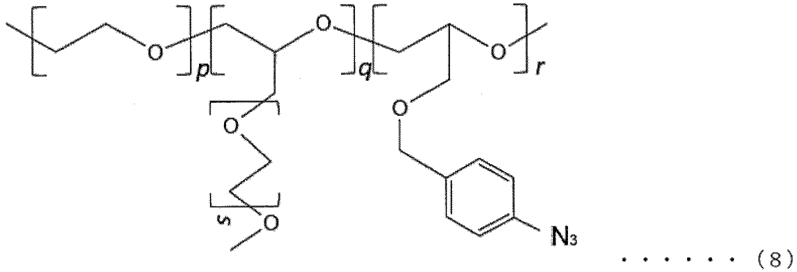

In formula (7), m is an integer of 10 or more and 1000 or less, and n is an integer of 10 or more and 1000 or less.

In formula (8), p is an integer of 10 or more and 1000 or less, q is an integer of 10 or more and 1000 or less, r is an integer of 10 or more and 1000 or less, and s is an integer of 10 or more and 1000 or less.

Preferable examples of the water-soluble polymer can also include water-soluble polymers each represented by the following general formula [I]. 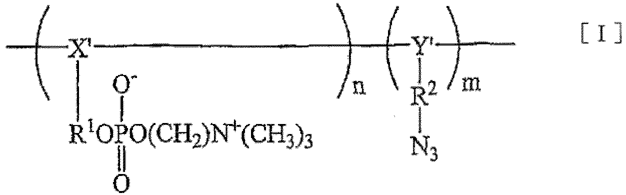 where: X', Y', R¹, and R² have the respective same meanings as defined above; n and m are each independently an integer of 2 or more, and n is greater than m; and units each containing X' and units each containing Y' are bonded in random order.

Among the compounds each represented by the above general formula [I], a polymer represented by the following formula [II] is particularly preferable. 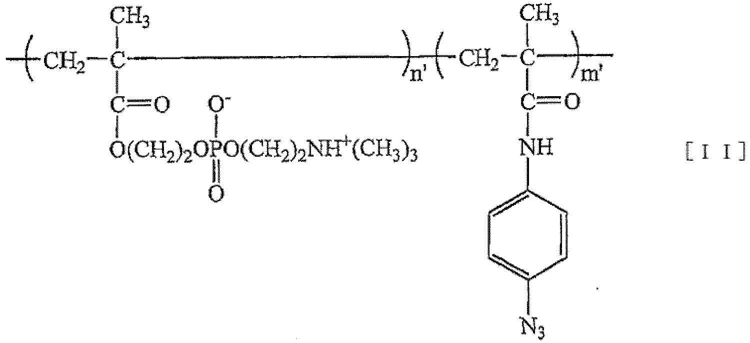 where: n' represents an integer of 50 to 200; m' represents an integer of 5 to 40; and phosphorylcholine-containing units and azidophenyl group-containing units are bonded in random order.

The water-soluble polymer can be easily carried out in accordance with the common technical knowledge of a person skilled in the art, and also examples of the water-soluble polymer are specifically described in Examples below.

### (Number of repeating units)

The number of repeating units each having the structure represented by formula (2) is preferably larger than the number of repeating units each having the structure represented by formula (1). The number of repeating units each having the structure represented by formula (4) is preferably larger than the number of repeating units each having the structure represented by formula (3). The number of the repeating units each having the structure represented by formula (1) or (3) is preferably 5 to 50, more preferably 10 to 40, and still more preferably 20 to 30, from the viewpoint that non-specific adsorption can be effectively prevented. The number of the repeating units each having the structure represented by formula (2) or (4) is preferably 50 to 200, more preferably 75 to 175, and still more preferably 100 to 150.

The water-soluble polymer is more preferably (i) a polymer constituted only by the structure represented by formula (1) and the structure represented by formula (2) or (ii) a polymer constituted only by the structure represented by formula (3) and the structure represented by formula (4). Note, however, that the water-soluble polymer may contain a unit derived from another polymerizable monomer, provided that the unit does not adversely affect the effect of the present invention. The proportion of such another unit is not particularly limited, provided that the proportion does not adversely affect the effect of the present invention. Usually, the proportion is not more than 30 mol%, and preferably not more than 10 mol%, with respect to the total units in the polymer.

The substance immobilizing agent (A) can be prepared by a known means. For example, with regard to the substance immobilizing agent (A), Japanese Patent Application Publication Tokukai No. 2020-132803 also describes some examples, and this description can be referred to as appropriate.

### [1-2. Example of substance immobilizing agent (B)]

The substance immobilizing agent (B) in accordance with another embodiment of the present invention is a substance immobilizing agent that contains a water-soluble synthetic polymer, a water-soluble naturally occurring polymer derivative, or the like each of which has, in one molecule thereof, at least two photoreactive groups. Examples of the photoreactive groups are the same as the examples of the photoreactive groups described in [1-1. Example of substance immobilizing agent (A)].

Examples of the water-soluble synthetic polymer include polyacrylic acids and derivatives thereof and polyvinyl alcohol and derivatives thereof. As described in [1-1. Example of substance immobilizing agent (A)], a polymer having a hydrophilic side chain (for example, a carboxylic acid group, an amino group, a hydroxyl group, or a halogen each having chemical reactivity) also exemplifies the water-soluble synthetic polymer. The photoreactive groups can be introduced into these polymers by a known chemical reaction. An example of introduction of the photoreactive groups into a polyacrylic acid has been reported: Biomaterials, 24, 3021-6 (2003). An example of introduction of the photoreactive groups into a polyvinyl alcohol has been reported: 2-(3-(4-azidophenyl)prop-2-enoylamino)-N-(4,4-dimethoxy-butyl)-3-(3-pyridyl)prop-2-enamide synthesized from 2-(2-(4-azidophenyl)vinyl)-4-(3-pyridylmethylene)-1,3-oxazolin-5-on; Biomaterials, 26, 211-216 (2005).

Introduction of the photoreactive groups into the main chain of the water-soluble polymer can be easily carried out in accordance with a conventional method. For example, by reacting a water-soluble polymer having a functional group with an azide compound having a functional group that reacts with the functional group, it is possible to bond an azide group to the water-soluble polymer. In a case where polyethylene glycol is used as the water-soluble polymer, polyethylene glycol having amino groups or carboxyl groups at both ends thereof is commercially available, and, therefore, by reacting an azide group-containing compound with a functional group of such a commercially available functional group-containing polyethylene glycol, it is possible to bond an azide group to the polyethylene glycol. A plurality of such methods are specifically described also in Examples below.

Examples of the water-soluble naturally occurring polymer can include, but are not limited to, naturally occurring polymers such as gelatin, casein, collagen, gum arabic, xanthan gum, tragacanth gum, guar gum, pullulan, pectin, sodium alginate, hyaluronic acid, chitosan, chitin derivatives, carrageenan, starches (carboxymethyl starch, aldehyde starch), dextrin, and cyclodextrin; and naturally occurring polymers such as water-soluble cellulose derivatives, e.g., methyl cellulose, viscose, hydroxyethyl cellulose, hydroxyethylmethyl cellulose, carboxymethyl cellulose, and hydroxypropyl cellulose. As an example of introduction of the photoreactive groups into a polysaccharide (hyaluronic acid), an example has been reported in which a synthesis is carried out by coupling azidoaniline to a hyaluronic acid via an amide group with use of a water-soluble carbodiimide: Macromol. Res., 21, 216-220 (2013). Further, introduction of the photoreactive groups into pullulan, which is another polysaccharide, has been also reported: Biomaterials, 26, 2401-2406 (2005). As an example of introduction of the photoreactive groups into gelatin, a synthetic example has been reported in which azidobenzoic acid is subjected to active esterification and a resultant active esterified azidobenzoic acid is coupled to gelatin via an amide group: Biotechnol. Bioeng., 2011 Oct; 108(10): 2468-76 (2011). It is possible to obtain the substance immobilizing agent by modifying, by a known method, each of these water-soluble polymers with a commercially available chemically-reactive and photoreactive crosslinking agent (for example, one that is manufactured by Thermo Fisher Scientific K.K.) via or by introduction of a carboxylic acid group, an amino group, a hydroxyl group, or a thiol group of the each of these water-soluble polymers.

### [1-3. Example of substance immobilizing agent (C)]

The substance immobilizing agent (C) in accordance with another embodiment of the present invention contains (i) a photocrosslinking agent which has, in one molecule thereof, at least two photoreactive groups and (ii) a water-soluble polymer of which the entire molecule is electrically neutral. Note, here, that the definition of the expression "entire molecule is electrically neutral" is the same as the definition described in [1-1. Example of substance immobilizing agent (A)].

The number of the photoreactive groups contained in one molecule of the photocrosslinking agent is, for example, 2 to 4, and preferably 2 to 3. Preferable examples of the photoreactive groups of the photocrosslinking agent can include, but are not limited to, azide groups (-N₃) and diazirines. The photocrosslinking agent is preferably a diazide compound or a diazirine compound each having two azide groups, and is particularly preferably a water-soluble diazide compound or a water-soluble diazirine compound. Preferable examples of the photocrosslinking agent used in an embodiment of the present invention can include diazide compounds each represented by the following general formula [III] or bisdiazirine compounds each represented by the following general formula [IV].

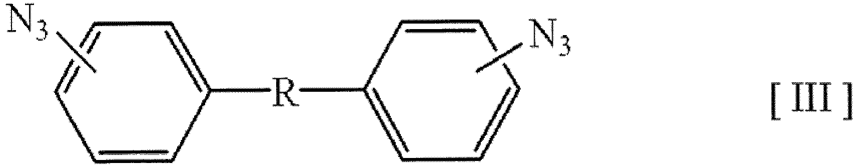

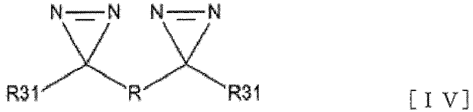

In general formulae [III] and [IV], R represents a single bond or any group. -R- is not particularly limited, because -R-is merely a structure for linking the at least two photoreactive groups. Examples of the photoreactive groups are the same as the examples of the photoreactive groups described in [1-1. Example of substance immobilizing agent (A)]. Preferable examples of -R- can include: a single bond (i.e., two phenyl azide groups are linked directly); C1-6 alkylene groups (in which one or two carbon-carbon unsaturated bonds may be contained or one or two carbon atoms may be double-bonded to oxygen to form a carbonyl group) (particularly preferably a methylene group); -CH₂-; -O-; -SO₂-; -S-S-; -S-; and - R2···Y···R3- (··· represents a single bond or a double bond, Y represents a C3-8 cycloalkylene group, R2 and R3 each independently represent a C1-6 alkylene group (in which one or two carbon-carbon unsaturated bonds may be contained, of which a carbon atom at an end may be double-bonded to Y, or in which one or two carbon atoms may be double-bonded to oxygen to form a carbonyl group), and, in the cycloalkylene group, substitution with any one or two or more substituents may be present (in a case where substitution is present, it is preferable that one or two of carbon atoms constituting the cycloalkylene group be double-bonded to oxygen to form a carbonyl group and/or substitution with one or two C1-6 alkyl groups is present)). Further, each of benzene rings in general formula [II] may have any one or two or more substituents (preferably, a hydrophilic group such as a halogen, a C1-4 alkoxyl group, or a sulfonic acid or a salt thereof). Further, each of the benzene rings in general formula [II] may have a structure having a medium molecular weight, such as polyoxyethylene. Specific preferable examples of -R- can also include the following.

In general formula [IV], the definition of R is the same as the definition of R in general formula [III]. R31 is F or CF₃.

Of photocrosslinking agents used in an embodiment of the present invention, water-soluble photocrosslinking agents are particularly preferable. The term "water-soluble" used with reference to a photocrosslinking agent means that it is possible to provide an aqueous solution having a concentration of not less than 0.5 mM, and preferably not less than 2 mM.

The concentration of the photocrosslinking agent in use (concentration before the photocrosslinking agent is mixed with a substance to be immobilized) is not particularly limited, but is preferably 1 µM to 2 mM, and more preferably 0.01 mM to 0.5 mM. The amount of the photocrosslinking agent added is not particularly limited, but is preferably 0.1 weight% to 50 weight%, particularly 1 weight% to 30 weight%, and more particularly 1 weight% to 10 weight%, with respect to the water-soluble polymer.

Preferable examples of the water-soluble polymer can include nonionic water-soluble polymers. Preferable examples etc. of the water-soluble polymer such as nonionic water-soluble polymers are the same as those described in [1-1. Example of substance immobilizing agent (A)].

In a case where a compound having both a hydrophilic group and a hydrophobic group is used as the photocrosslinking agent, it is preferable that the water-soluble polymer be a copolymer which contains (i) a unit having a phosphobetaine structure and (ii) a constitutional unit, such as (meth)acrylate alkyl ester (preferably, the number of carbon atoms in an alkyl moiety is 3 to 6), derived from a monomer containing both a hydrophilic group and a hydrophobic group (alkyl ester moiety), in order to prevent micellization. Further, a water-soluble polymer having a plurality of photoreactive groups described in [1-1. Example of substance immobilizing agent (A)] or [1-2. Example of substance immobilizing agent (B)] can be mixed.

With regard to the substance immobilizing agent (C), the pamphlet of International Publication No. WO 2005/111618 also describes some examples thereof, and this description can be referred to as appropriate.

### [1-4. Example of substance immobilizing agent (D)]

The substance immobilizing agent (D) in accordance with another embodiment of the present invention contains (i) a water-soluble monomer of which the entire molecule is electrically neutral and which has a polymerizable group (hereinafter also simply referred to as "water-soluble monomer" or "monomer"), (ii) a photopolymerization initiator, and (iii) a photocrosslinking agent which has, in one molecule thereof, at least two photoreactive groups. Note, here, that the definition of the expression "entire molecule is electrically neutral" is the same as the definition described in [1-1. Example of substance immobilizing agent (A)].

The substance immobilizing agent in accordance with an embodiment of the present invention can cause, by photo-irradiation, two kinds of reactions: (1) polymerization of the monomer; and (2) radicalization of the photoreactive groups contained in the photocrosslinking agent. As a result, a polymer is formed by the polymerization of the monomer, and, by bonding the polymer thus formed to a substrate via the photocrosslinking agent and bonding the polymer to a substance to be immobilized via the photocrosslinking agent, it is possible to ultimately immobilize the substance to the substrate. Since the entire molecule of the monomer contained in the substance immobilizing agent in accordance with an embodiment of the present invention is electrically neutral, an electrically neutral polymer can be formed on the substrate by the polymerization of the monomer. The polymer thus formed can exhibit a non-specific adsorption preventing effect on the substrate. Thus, according to an embodiment of the present invention, it is possible to immobilize a desired substance to a substrate while preventing non-specific adsorption. A method for immobilizing a substance to a substrate will be described later.

The following description will discuss the monomer, the photopolymerization initiator, and the photocrosslinking agent contained in the substance immobilizing agent in accordance with an embodiment of the present invention.

### (Water-soluble monomer)

The monomer of which the entire molecule is electrically neutral can be an amphoteric or nonionic monomer. Note, here, that the term "amphoteric" means having groups which are ionized in an aqueous solution having a pH in the vicinity of neutral (e.g., pH 6 to 8) to respectively become a cation and an anion such that the total of electric charges in one molecule of a monomer is substantially 0 (zero). The definition of the term "nonionic" is the same as the definition described in [1-2. Example of substance immobilizing agent (B)]. In particular, the nonionic monomer has an excellent non-specific adsorption preventing effect, and has an advantage of being inexpensively produced or obtained.

Specific examples of the water-soluble monomer are the monomers listed as preferable monomers of the nonionic water-soluble polymer described in [1-2. Example of substance immobilizing agent (B)].

Further, preferable examples of the water-soluble monomer can include phosphoryl-containing amphoteric monomers each having the structure represented by general formula [I] described in [1-2. Example of substance immobilizing agent (B)].

The concentration of the water-soluble monomer in the substance immobilizing agent in accordance with an embodiment of the present invention can be 0.0001 mass% to 10 mass%, and preferably 0.001 mass% to 1 mass%.

### (Photopolymerization initiator)

The substance immobilizing agent (D) in accordance with an embodiment of the present invention contains the photopolymerization initiator together with the water-soluble monomer. The photopolymerization initiator generates a radical (active species) by energy by photo-irradiation, e.g., irradiation with ultraviolet rays. The radical reacts with the polymerizable group of the monomer to initiate the polymerization. As the photopolymerization initiator, a benzophenone-based photopolymerization initiator, a benzoin-based photopolymerization initiator, an acetophenone-based photopolymerization initiator, or a thioxanthone-based photopolymerization initiator can be used. Since the properties, such as ultraviolet ray absorbing properties, a reaction initiating efficiency, and yellowing, of the photopolymerization initiator vary depending on the kind of the photopolymerization initiator, it is preferable to select a suitable photopolymerization initiator in consideration of these properties. Further, an auxiliary agent referred to as a sensitizer can be also used in combination so as to promote an initiation reaction of the photopolymerization initiator.

The substance immobilizing agent (D) in accordance with an embodiment of the present invention preferably contains a water-soluble photopolymerization initiator. Specifically, the substance immobilizing agent (D) preferably contains 1-[4-(2-hydroxyethoxy) -phenyl] -2-hydroxy-2-methyl-1-propan-1-on, (4-benzoylbenzyl)trimethylammonium chloride, or the like.

The amount of the photopolymerization initiator added is not particularly limited, but is preferably 1 mass% to 20 mass%, and more preferably 2 mass% to 10 mass%, with respect to the monomer.

### (Photocrosslinking agent)

For example, the same photocrosslinking agent as included in the substance immobilizing agent (C) can be used. Note that, with regard to the substance immobilizing agent (D), Japanese Patent Application Publication, Tokukai, No. 2006-316010 also describes some examples thereof, and this description can be referred to as appropriate.

### [2. Substance to be immobilized]

A substance to be immobilized with use of a substance immobilizing agent in accordance with an embodiment of the present invention is not particularly limited. Examples of the substance can include polypeptides (including glycoproteins and lipoproteins), enzymes, polysaccharides, nucleic acids, lipids, cells (such as animal cells, plant cells, and microorganism cells), and components (including cell organelles, such as nuclei and mitochondria, and membranes, such as cell membranes and unit membranes) of the cells. The other examples of the substance can include allergens, antigens such as autoantigens, and viruses. When photoreactive groups (in particular, an azide group and/or a diazirynyl group) contained in the substance immobilizing agent in accordance with an embodiment of the present invention are irradiated with light, a nitrogen molecule is detached and nitrin and/or carbene is generated. The nitrin and/or the carbene can be bonded not only to a functional group such as an amino group or a carboxyl group but also to a carbon atom constituting an organic compound. Therefore, it is possible to immobilize most organic substances without orientation.

### [3. Substrate]

A substrate is not particularly limited, provided that at least a surface of the substrate is made of a substance which can be bonded to photoreactive groups described above. Examples of the substrate can include substrates made of organic substances such as polystyrene, which is widely used in microplates and the like, polyethylene terephthalate, ABS resin, polycarbonate, and polypropylene. A glass plate coated with a silane coupling agent can also be, for example, used. The form of the substrate is not limited in any way, and a platelike substrate, such as a substrate for a microarray, a bead-like substrate, a fiber-like substrate, or the like can be used. Furthermore, a hole or a groove provided in a plate (e.g., a well of a microplate) can also be, for example, used. A substance immobilizing agent in accordance with an embodiment of the present invention is particularly suitable for a microarray among these.

The surface of the substrate, to which surface the substance is immobilized, may be modified. Examples of modification include causing the surface of the substrate to be moderately hydrophilic by a hydrophilization treatment such as a UV-ozone treatment, a plasma treatment, a glow discharge treatment, and a corona discharge treatment. In a case where the hydrophilicity of the surface of the substrate is expressed by a contact angle with water, the contact angle preferably falls within a range of not less than 20° and less than 80°. The contact angle falls within a range of more preferably not less than 25° and less than 60°, not less than 25° and not more than 55°, or not less than 35° and not more than 55°, and particularly preferably not less than 38° and not more than 51° The contact angle with water is measured in the following manner with use of DropMaster500 manufactured by Kyowa Interface Science Co., Ltd.: 1 µL of distilled water is dropped onto the surface of the substrate, and then a static contact angle is measured after 1000 ms. An analysis method is a 0/2 method (see also Examples). In a case where the contact angle between the surface of the substrate and water falls within the above ranges, 1) it is easier to uniformly coat the surface of the substrate with the substance immobilizing agent and 2) it is easier to control the diameter of a spot which is formed on a layer of the substance immobilizing agent and which contains the substance to fall within an appropriate range.

### [4. Method for immobilizing substance]

### (1) Immobilizing method in accordance with first embodiment

A method for immobilizing a substance in accordance with an embodiment of the present invention (hereinafter, sometimes abbreviated as "substance immobilizing method") includes: coating a surface of a substrate with an aqueous solution or an aqueous suspension each of which contains a substance immobilizing agent described above and a substance to be immobilized to the substrate; and photo-irradiating the surface of the substrate which surface has been coated with the aqueous solution or the aqueous suspension.

In the case of the substance immobilizing agent (A) or (B), the concentration (wt/vol%) of the water-soluble polymer in the aqueous solution or the aqueous suspension is not particularly limited, and is, for example, approximately 0.005% to 10%, preferably approximately 0.05% to 5%. The concentration is more preferably approximately 0.5% to 3%.

A method for coating the surface of the substrate with the aqueous solution or the aqueous suspension, each of which contains the substance immobilizing agent, is not particularly limited, and examples thereof include a spin coating method, a spray coating method, and a dip method.

Next, irradiation with light is carried out, preferably after the aqueous solution or the aqueous suspension with which the surface of the substrate is coated has been dried. The light is light which enables photoreactive groups used to generate radicals. In a case where an azide group and/or a diazirynyl group are/is used as the photoreactive groups, ultraviolet rays are preferable. The dose of a light beam with which irradiation is carried out is not particularly limited, and is usually approximately 1 mW to 100 mW per square centimeter.

In the case of the substance immobilizing agent (A) or (B), the photoreactive groups in the polymer generate radicals by irradiation with light, and the polymer is covalently bonded to both the substrate and the substance to be immobilized. In the case of the substance immobilizing agent (C), the photoreactive groups in the photocrosslinking agent generate radicals by irradiation with light, and the polymer is covalently bonded to both the substrate and the substance to be immobilized via the photocrosslinking agent. In the case of the substance immobilizing agent (D), the water-soluble monomer is polymerized to form a polymer, by irradiation with light. Furthermore, the photoreactive groups in the photocrosslinking agent generate radicals, and the polymer is covalently bonded to both the substrate and the substance to be immobilized via the photocrosslinking agent. As a result, the substance to be immobilized is immobilized to the substrate via the polymer.

In the substance immobilizing method, a bonding reaction is carried out with use of the radicals generated by the photoreactive groups. Therefore, the polymer is bonded not to a specific site but to a random site of the substance to be immobilized. Therefore, it is considered that a molecule of which an active site is subjected to bonding and which accordingly loses activity also obviously appears. On the other hand, a molecule which is bonded at a site that does not affect an active site is also obviously present. Thus, according to the substance immobilizing method, even in a case where the substance is one that has been conventionally difficult to covalently immobilize due to the presence of an appropriate substituent at or near an active site, it is possible to covalently immobilize the substance to the substrate without loss of activity as a whole.

At a part of the substrate which part has not been irradiated with light, the photoreactive groups are not bonded to the substrate or the substance. Accordingly, when washed, the polymer, the monomer (in the case of the substance immobilizing agent (D)), and the substance are removed at such a part. Thus, it is possible to immobilize the substance in any pattern by carrying out selective exposure via a photomask or the like. Therefore, the substance immobilizing method is very advantageous because it is possible to immobilize the substance in any of various shapes, such as the shape of a microarray, by selective exposure.

### (2) Immobilizing method in accordance with second embodiment

Alternatively, the following method may be employed: a substrate is first coated with a substance immobilizing agent in accordance with an embodiment of the present invention; a substance to be immobilized is placed on the substance immobilizing agent by microspotting; and then an entire surface of the substrate is photo-irradiated. In this case, a spot of the substance to be immobilized is formed on a layer of the substance immobilizing agent, and the proportion of the substance that is immobilized to the substrate by a covalent bond via the substance immobilizing agent is increased. The substrate may be coated with the substance immobilizing agent in the following manner, as in the above "Immobilizing method in accordance with first embodiment": the surface of the substrate is coated with an aqueous solution or an aqueous suspension each of which contains the substance immobilizing agent. The condition of the concentration of the water-soluble polymer or the water-soluble monomer each of which is contained in the aqueous solution or the aqueous suspension only needs to be the same as that in the above "Immobilizing method in accordance with first embodiment".

Alternatively, the following method may be employed: the substance immobilizing agent in accordance with an embodiment of the present invention is placed on the substrate by microspotting; the substance to be immobilized is placed on the substance immobilizing agent by microspotting; and then the entire surface of the substrate is photo-irradiated. Also in this case, a spot of the substance to be immobilized is formed on a layer (separate spot) of the substance immobilizing agent, and the proportion of the substance that is immobilized to the substrate by a covalent bond via the substance immobilizing agent is increased.

### (3) Immobilizing method in accordance with third embodiment

Alternatively, the following substance immobilizing method may be employed: a mixture of a substance immobilizing agent in accordance with an embodiment of the present invention and a substance to be immobilized is placed on a substrate by microspotting; and then an entire surface of the substrate is photo-irradiated. Microspotting is a method for coating, with a liquid, an extremely narrow area on a substrate, and is commonly used to prepare a DNA chip and the like. Devices for microspotting are also commercially available. Therefore, microspotting can be easily carried out with use of a commercially available device.

In a case where microspotting is carried out as in the immobilizing method in accordance with the second or third embodiment, a solution to be placed by spotting can contain a thickener. By using the thickener, effects such as stabilization of the droplet size of a microspot and a decrease in a CV value (coefficient of variation between spots) can be expected. Examples of the thickener include: ethylene glycol, polyethylene glycol, polyvinyl alcohol, glycerin, and carboxymethyl cellulose, and ethylene glycol, polyethylene glycol, or polyvinyl alcohol is preferable.

The concentration of the thickener in the solution to be placed by spotting is not particularly limited, and may be determined, as appropriate, so as to achieve a desired droplet size. In a case where the thickener is ethylene glycol, the concentration falls within a range of preferably 3 weight% to 75 weight%, more preferably 4 weight% to 60 weight%, and still more preferably 5 weight% to 50 weight%.

The substance immobilizing method can be suitably used to prepare a plate for an immunoassay (including an allergen chip) to which plate an antibody, an antigen-binding fragment thereof, or an antigen is immobilized and to prepare a nucleic acid chip, a microarray, and the like in each of which DNA or RNA is immobilized to a substrate. However, the substance immobilizing method is not limited to preparation of these. For example, the substance immobilizing method can also be applied to immobilization of an entire cell or a component thereof.

### [5. Substrate coated with substance immobilizing agent]

A substrate in accordance with an embodiment of the present invention has a surface to which a substance is to be immobilized and on which a layer of a substance immobilizing agent is formed. By placing, on the layer of the substance immobilizing agent, the substance to be immobilized and carrying out photo-irradiation, it is possible to immobilize the substance to the substrate via the layer of the substance immobilizing agent.

The thickness of the layer of the substance immobilizing agent on the substrate falls within a range of not less than 3 nm and less than 500 nm, preferably not less than 3 nm and not more than 450 nm, more preferably not less than 3 nm and not more than 350 nm, and still more preferably not less than 3 nm and not more than 300 nm. Note that the thickness of the layer of the substance immobilizing agent may be the thickness of the layer before the substance is immobilized or the thickness of the layer after the substance has been immobilized. In a case where the thickness of the layer of the substance immobilizing agent falls within a given range, a sufficient signal can be obtained when the substrate which is coated with the substance immobilizing agent is used for testing or the like (described later). Moreover, it is possible to suppress non-specific adsorption to the substrate.

The thickness of the layer of the substance immobilizing agent can be determined with use of, for example, a spectroscopic ellipsometer. With respect to data (Ψ and Δ) obtained with use of the spectroscopic ellipsometer, fitting is carrying out with use of a Cauchy model to determine each of the thickness of the substrate before the layer of the substance immobilizing agent is formed thereon and the thickness of the substrate after the layer of the substance immobilizing agent has been formed thereon. A difference between the former and the latter is the thickness of the layer of the substance immobilizing agent. It is also possible to refer to the description in Examples.

### [6. Kit for testing and method for testing]

The present invention also provides a kit for testing which kit includes a substrate to which a substance is immobilized. The kit for testing may further include instructions for use of the kit, as necessary. In a case where the substance immobilized to the substrate is an allergen, the kit can be a kit for allergy testing or antibody testing. In a case where the substance immobilized to the substrate is an antibody, the kit can be a kit for biomarker testing by a sandwich method. The present invention also provides a method for testing an interaction between (i) a substance immobilized to a substrate and (ii) a sample, with use of the substrate to which the substance is immobilized or the kit for testing. An example of the sample is a sample derived from a living body. Examples of such a sample include samples derived from body fluids such as blood (including some components, such as a serum, of blood), saliva, and cerebrospinal fluid.

A signal of the interaction between (i) the substance immobilized to the substrate and (ii) the sample may be detected by a method which varies depending on the kind of the signal, e.g., a chemiluminescence method or a fluorescent method.

### [7. Summary of embodiments]

The embodiments of the present invention include, for example, the following aspects.
1) A substrate that has a surface to which a substance is to be immobilized and on which a layer of a substance immobilizing agent is formed, wherein the substance immobilizing agent has photoreactive groups and contains a water-soluble polymer or a water-soluble monomer which turns into the water-soluble polymer by polymerization, and a thickness of the layer of the substance immobilizing agent is not less than 3 nm and less than 500 nm.
2) The substrate as described in 1), wherein the substance immobilizing agent is one of the following (A), (B), (C), and (D): (A) the water-soluble polymer is constituted by a polymer which has, in one molecule thereof, at least two photoreactive groups and of which molecule is electrically neutral or the water-soluble polymer has a main chain which contains a polyoxyethylene structure or a polyoxyalkylene structure; (B) the water-soluble polymer is a water-soluble synthetic polymer or a water-soluble naturally occurring polymer derivative each of which has, in one molecule thereof, at least two photoreactive groups; (C) the substance immobilizing agent contains (i) a photocrosslinking agent which has, in one molecule thereof, at least two photoreactive groups and (ii) the water-soluble polymer of which molecule is electrically neutral; and (D) the substance immobilizing agent contains (i) the water-soluble monomer of which molecule is electrically neutral and which has a polymerizable group, (ii) a photopolymerization initiator, and (iii) a photocrosslinking agent which has, in one molecule thereof, at least two photoreactive groups.
3) The substrate as described in 1) or 2), to which the substance is immobilized via the layer of the substance immobilizing agent.
4) The substrate as described in 3), to which the substance is immobilized.
5) The substrate as described in any one of 1) to 4), wherein the substance is selected from the group consisting of polypeptides, enzymes, polysaccharides, nucleic acids, lipids, cells, and components of the cells.
6) The substrate as described in any one of 2) to 5), wherein the substance immobilizing agent is one of the following (b), (c), and (d): (b) the water-soluble polymer is constituted by a nonionic water-soluble polymer which has, in one molecule thereof, at least two photoreactive groups, and the nonionic water-soluble polymer is polyalkylene glycol or a polyvinyl-based polymer; (c) the substance immobilizing agent contains (i) a photocrosslinking agent which has, in one molecule thereof, at least two photoreactive groups and (ii) the water-soluble polymer of which molecule is electrically neutral; and (d) the substance immobilizing agent contains (i) polyethylene glycol (meth)acrylate as the water-soluble monomer, (ii) a photopolymerization initiator, and (iii) a photocrosslinking agent which has, in one molecule thereof, at least two azide groups as the photoreactive groups.
7) The substrate as described in any one of 1) to 6), wherein a contact angle with water of the surface of the substrate, to which surface the substance is to be immobilized, is not less than 20° and less than 80°.
8) The substrate as described in any one of 1) to 7), wherein the photoreactive groups are contained in an amount of not less than 2.5 mol% and less than 50 mol% with respect to the entire water-soluble polymer.
9) A kit for allergy testing or antibody testing, including the substrate described in any one of 1) to 8), wherein the substance is an allergen.
10) A kit for biomarker testing by a sandwich method, including the substrate described in any one of 1) to 8), wherein the substance is an antibody.
11) A method for testing, with use of the substrate described in any one of 1) to 8) or the kit described in 9) or 10), an interaction between (i) the substance immobilized to the substrate and (ii) a sample.

With reference to Examples below, the embodiments of the present invention will be described in more detail. Obviously, the present invention is not limited to the following Examples, and it is needless to say that various aspects are employed for details. Furthermore, the present invention is not limited to the above-described embodiments, and various modifications can be made within the scope of the claims. The present invention encompasses, in its technical scope, an embodiment obtained by appropriately combining technical means disclosed in differing embodiments. Further, all of the documents listed herein are incorporated herein by reference.

### Examples

In the following Examples, % represents mass% unless otherwise specified.

### [Example 1]

### (1) Materials etc.

### = Substrate =

A black substrate made of an ABS resin (ABS-B-050501, manufactured by AS ONE CORPORATION) or a quartz substrate (3-131-601, manufactured by Kenis) was used. The thickness of each of these substrates was 1 mm. Each of the substrates was cut so as to have a size of 23 mm × 26 mm, and was used in an experiment below.

### = Coating agent (substance immobilizing agent) =

The following three kinds of coating agents were prepared.

### · Multivalent crosslinking type (corresponding to the substance immobilizing agent (A))

This coating agent is also referred to as polyvinyl type Az-PEG-1. The coating agent was produced by a method described in Example 1 of Japanese Patent No. 4630817 (International Publication No. WO 2004/088319). That is, in an eggplant type flask, N-azidophenylacrylamide and PEG (degree of polymerization n=8) monomethacrylate were dissolved in ethyl acetate. Azobisisobutyronitrile (AIBN) was then added to a resultant solution. After the flask was filled with nitrogen and stoppered tightly, a resultant mixture was stirred at 60°C for 6 hours. After heating was stopped and the solvent was evaporated to some degree, a resultant mixture was transferred to diethyl ether. A resultant mixture was subjected to a sonicator. A solid was collected and dried under reduced pressure for 2 hours. As a result, a photoreactive poly(ethylene glycol monomethacrylate-N-azidophenylacrylamide) copolymer was obtained. Az-PEG-1 is a photoreactive and water-soluble polymer containing 10 mol% of an azidophenyl group-containing monomer.

### · Multivalent crosslinking type (corresponding to the substance immobilizing agent (A))

This coating agent is also referred to as oxyethylene type Az-PEG-2. The coating agent was produced by a method described in Example 1 of Japanese Patent Application Publication Tokukai No. 2020-132803. Az-PEG-2 is a photoreactive and water-soluble polymer containing not less than 2.5 mol% and less than 50 mol% of an azidophenyl group-containing monomer.

### · Photoreaction addition type (corresponding to the substance immobilizing agent (B))

Photoreactive hyaluronic acid (Hyal-Az) was synthesized in accordance with a method described in Macromol. Re., 21, 216 (2013) DOI 10.1007/s13233-013-1016-7. Sodium hyaluronate (100 mg) was dissolved in 100 mL of water. To a resultant solution, 1-ethyl-3-(3-dimethylaminopropyl)carbodiimide hydrochloride (92 mg) and 4-azidoaniline hydrochloride (99 mg) were added. A resultant mixture was reacted at room temperature for 24 hours while being stirred at pH 7.0 in a dark room. Thereafter, purification was carried out by dialysis to obtain photoreactive hyaluronic acid. The photoreactive hyaluronic acid contains 10 mol% of an azidophenyl group-containing saccharide monomer.

### · Crosslinking agent mixed type (corresponding to the substance immobilizing agent (C))

This coating agent is also referred to as mixed type Az-1. Poly(ethylene glycol) (average molecular weight: 9501050, Ardrich) was used as a polymer, and 4,4'-diazidostilbene-2,2'-disulfonate (Fluka) was used as a photocrosslinking agent. A substance immobilizing agent (C) was prepared by dissolving the polymer and the crosslinking agent in an aqueous solution (water:ethanol = 50 vol%:50 vol%) so that the concentration of the polymer became 9% and the concentration of the crosslinking agent became 1%.

### (2) Method for coating substrate with coating agent

Glow discharge (100 w, 90 seconds) was carried out with use of Ipsolon (Izumi Kogyo) with respect to the ABS substrate or the quartz substrate, each of which had been cut so as to have a size of 23 mm × 26 mm. After the glow discharge, spin coating was carried out with use of a spin coater (MS-B100, manufactured by MIKASA) in the following manner: aqueous solutions (water:ethanol = 50 vol%:50 vol%) which contained the respective above coating agents at different concentrations were prepared; 70 µL of each of the aqueous solutions was dropped; and then spin coating was carried out while the rotational speed was changed.

### (3) Measurement of coating thickness of coating agent

The coating thickness of the coating agent was measured in the following manner by ellipsometry.

### [Measuring device]

A spectroscopic ellipsometer (M-2000UI, manufactured by J. A. Woollam, Co., Inc., Lincoln, NE, USA) was used.

### [Measurement conditions and measurement procedure]

Measurement was carried out in the vicinity of the center of the ABS substrate before the ABS substrate was coated with the coating agent, under the following conditions: the range of a measurement wavelength was 250 nm to 1700 nm; angles at which light was incident on a sample were 50°, 60°, and 70°; and a data-accumulation count was 100. With respect to data (Ψ and Δ) obtained by the ellipsometry, fitting was carried out with use of a Cauchy model to determine the refractive index and the thickness of the ABS substrate before the ABS substrate was coated with the coating agent. The Cauchy model is a phenomenological model in which only normal distribution of a refractive index in a transparent dielectric material is considered. However, since a good fitting result was obtained, the extinction coefficient k of the black ABS substrate was considered to be negligibly small, and in an analysis, a result obtained by approximation with k=0 (neglecting light absorption) was used. A refractive index in the Cauchy model is expressed in the form of n(λ)=A+B/λ²+C/λ⁴ (where λ is a wavelength of light incident on a sample). Coefficients A, B, and C were determined by fitting.

After the ABS substrate before the ABS substrate was coated with the coating agent was evaluated, thin coating of the coating agent was formed, and then measurement and an analysis of the ABS substrate which had been vacuum-dried were carried out. As an analytical model, a structure in which a single layer of the coating agent was laid on the ABS substrate was assumed. As the refractive index of the ABS substrate itself, the above result obtained with respect to the ABS substrate before the ABS substrate was coated with the coating agent was used. The coating thickness of the layer of the coating agent was determined by fitting the refractive index (coefficient of the Cauchy model).

In general, in a case where coating is thin (approximately not more than 10 nm), it is difficult to accurately determine the coating thickness and the refractive index thereof independently. For this reason, in many cases, thick coating is used to determine the refractive index thereof, and then thin coating is used to determine only the coating thickness thereof by fitting. In the present Example, since there was a possibility that the refractive index would vary as the amount of a remaining solvent varied depending on the conditions under which the substance immobilizing agent was dried, the refractive index was analyzed also by fitting.

As for the quartz substrate, the reproducibility of the refractive index thereof was extremely high, and it was possible to confirm that a measurement result of the quartz substrate alone used in this measurement sufficiently matched a value indicated by analysis software of the ellipsometer.

Then, it was confirmed that, by keeping the concentration of the coating agent and the rotational speed of the spin coating constant, similar coating thicknesses were achieved on both the quartz substrate and the ABS resin. The relationship between the conditions of coating formation and the coating thicknesses was determined and used as a reference.

Furthermore, it was confirmed that these coating thicknesses obtained with use of the ellipsometer were comparable with those measured with use of a reflective confocal laser microscope (OLS4100, manufactured by Olympus).

### (4) Method for measuring emission intensity

A spot solution containing 100 µg/mL of a protein [biotinylated BSA (manufactured by Nippon Chemiphar Co., Ltd., and obtained by labeling BSA with biotin with use of a biotin labeling reagent (Biotin Sulfo-OSu, product code: B319, manufactured by DOJINDO)) or rabbit IgG (manufactured by Sigma)] was prepared, and was micro-spotted, with use of a pin arrayer (MicroGrid II compact, manufactured by BioRobotics), on the ABS substrate which had been coated with the aqueous solution of the coating agent by spin coating.

The substrate on which microspots had been arrayed was dried in a vacuum dryer for 10 minutes, and then irradiated with UV rays with use of a UV irradiation device (UV Crosslinker: UVP CL1000S, 254 nm UV) for 10 minutes so that the microspots were photo-immobilized.

The substrate was then pre-washed with 200 µL of PBS for 10 seconds. This pre-washing was carried out three times in total. Subsequently, 120 µL of 3000-fold diluted Streptavidin Alkaline Phosphatase or a labeled antibody (manufactured by Promega) was reacted with the photo-immobilized protein for 8 minutes. After the reaction, the substrate was washed with 200 µL of PBS for 10 seconds. This washing was carried out six times in total. Then, 120 µL of a substrate (DynaLight, manufactured by Thermo Fisher) (stock solution) was applied to the substrate. The amount of light emitted after 30 seconds of application was measured with use of a CCD camera. By counting the number of pixels with use of SpotSolver manufactured by Dynacom Co., Ltd. while regarding a non-immobilized area as a background and an immobilized area as a signal, emission intensity was measured. Measurement results of backgrounds are shown in Tables 1 to 4, and measurement results of signals are shown in Tables 5 to 8.

**[Table 1]**

| Background, AzPEG-1, ABS substrate, Biotinylated BSA | | |
|---|---|---|
| Coating thickness (nm) | Emission intensity (pixel) | % |
| 0 | 100,367 | 100 |
| 1 | 52,235 | 52.0 |
| 3 | 10,530 | 10.5 |
| 6 | 11,745 | 11.7 |
| 10 | 8,666 | 8.6 |
| 14 | 8,072 | 8.0 |

**[Table 2]**

| Background, AzPEG-1, Quartz substrate, Biotinylated BSA | | |
|---|---|---|
| Coating thickness (nm) | Emission intensity (pixel) | % |
| 0 | 32,270 | 100 |
| 1.3 | 21,310 | 66.0 |
| 2.7 | 16,424 | 50.9 |
| 3.9 | 4,219 | 13.1 |
| 6.4 | 3,075 | 9.5 |
| 13.8 | 2,329 | 7.2 |
| 17.2 | 2,346 | 7.3 |

**[Table 3]**

| Background, Hyal-Az, ABS substrate, Rabbit IgG | | |
|---|---|---|
| Coating thickness (nm) | Emission intensity (pixel) | % |
| 0 | 167,683 | 100 |
| 3 | 13,269 | 7.9 |
| 6 | 3.733 | 2.2 |
| 10 | 3,061 | 1.8 |
| 30 | 2,618 | 1.6 |

**[Table 4]**

| Background, Mixed type Az-1, ABS substrate, Rabbit IgG | | |
|---|---|---|
| Coating thickness (nm) | Emission intensity (pixel) | % |
| 0 | 100,400 | 100 |
| 3 | 480 | 0.5 |
| 6 | 3,200 | 3.2 |
| 10 | 1,504 | 1.5 |
| 30 | 1,581 | 1.6 |

**[Table 5]**

| Signal, AzPEG-1, ABS substrate, Biotinylated BSA | | |
|---|---|---|
| Coating thickness (nm) | Emission intensity (pixel) | % |
| 141 | 432,726 | 94.5 |
| 216 | 458,138 | 100 |
| 328 | 393,771 | 86.0 |
| 678 | 48,657 | 10.6 |

**[Table 6]**

| Signal, AzPEG-2, ABS substrate, Rabbit IgG | | |
|---|---|---|
| Coating thickness (nm) | Emission intensity (pixel) | % |
| 100 | 313,170 | 91.0 |
| 300 | 344,170 | 100.0 |
| 500 | 5,449 | 1.6 |

**[Table 7]**

| Signal, Hyal-Az, ABS substrate, Rabbit IgG | | |
|---|---|---|
| Coating thickness (nm) | Emission intensity (pixel) | % |
| 30 | 163,729 | 100 |
| 100 | 122,909 | 75.1 |
| 300 | 138,030 | 84.3 |
| 400 | 38,548 | 23.5 |
| 500 | 1,504 | 0.9 |

**[Table 8]**

| Signal, Mixed type Az-1, ABS substrate, Biotinylated BSA | | |
|---|---|---|
| Coating thickness (nm) | Emission intensity (pixel) | % |
| 30 | 143,025 | 114.9 |
| 300 | 124,500 | 100.0 |
| 500 | 1,291 | 1.0 |

In order to obtain signal intensity with a sufficiently high S/N ratio, it is necessary to reduce noise (background) as much as possible. The minimum coating thickness required for this purpose was determined. From Tables 1 to 4, it was found that a coating thickness of not less than 3 nm enabled non-specific adsorption to be sufficiently suppressed, i.e., suppressed to not more than 15% as compared with an uncoated state.

In order to obtain signal intensity with a sufficiently high S/N ratio, it is necessary to increase an immobilized protein as much as possible. However, although the amount of an immobilized protein is increased as a coating thickness is increased, the surface concentration of a biopolymer (protein) for interacting with an analyte on the outermost surface is limited, and protein interaction is suppressed. An optimum coating thickness is determined depending on the concentration of a protein added. From Tables 5 to 8, it was found that, at a typical protein concentration, emission intensity sharply decreased when the coating thickness was 500 nm. Therefore, it was found that, in order to obtain signal intensity with a sufficiently high S/N ratio, the layer of the substance immobilizing agent needed to have a thickness of not less than 3 nm and less than 500 nm, irrespective of a material of a substrate. In particular, it was found that the layer of the substance immobilizing agent particularly preferably had a thickness falling within a range of 3 nm to 350 nm.

### [Example 2]

In the same manner as in Example 1, an interaction between a protein and a labeled antibody was detected. Note, however, that, instead of the glow discharge, a plasma treatment was carried out with use of a plasma treatment device (Plasma Cleaner PC-300, manufactured by Samco). A contact angle with water was measured on a surface of an ABS substrate before the ABS substrate was coated with a coating agent, by various plasma treatments. As the coating agent, oxyethylene type Az-PEG-2 and polyvinyl type Az-PEG-1 (substance immobilizing agent (A)) were each used, and the concentration thereof was 1.0 wt/vol%. Coating formation was carried out under the conditions under which a coating thickness of 10 nm was achieved.

The contact angle (static contact angle) with water was measured as follows. As a measurement device, DropMaster500 manufactured by Kyowa Interface Science Co., Ltd. was used. An analysis method was a θ/2 method. Onto the surface of the ABS substrate, 1 µL of distilled water was dropped. A time period from dropping to measurement was 1000 ms. Measurement results are shown in Tables 9 and 10.

In Table 9, the "Amount of azidophenyl group" indicates the amount (mol%) of an azidophenyl group contained in the substance immobilizing agent. In the "Kind of substance immobilizing agent", "1" indicates Az-PEG-1, and "2" indicates Az-PEG-2.

**[Table 9]**

| Cases where polyvinyl type Az-PEG-1 or Az-PEG-2 (substance immobilizing agent (A)) was used | | | | |
|---|---|---|---|---|
| Amount of azidophenyl group | Kind of substance immobilizing agent | Solubility in volatile polar solvent | Signal emission intensity (pixel) | Signal emission intensity (%) |
| 2.5 | 2 | ○ | 45,399 | 21 |
| 5.0 | 2 | ○ | 100,768 | 48 |
| 10.0 | 2 | ○ | 132,219 | 63 |
| 10.0 | 1 | ○ | 125,269 | 60 |
| 15.0 | 2 | ○ | 156,854 | 75 |
| 20.0 | 2 | ○ | 169,674 | 81 |
| 30.0 | 2 | ○ | 209,138 | 100 |
| 40.0 | 2 | ○ | 151,979 | 72 |
| 50.0 | 2 | × | - | - |

**[Table 10]**

| Cases where polyvinyl type Az-PEG-1 (substance immobilizing agent (A)) was used | | | |
|---|---|---|---|
| Plasma treatment | Contact angle (°) | Diameter of spot (referece: 2.25 mm) | Emission intensity (%) |
| No treatment | 80 | Uniform coating was impossible | |
| 10W 2s | 51 | Smaller than reference | 95 |
| 10W 5s | 46 | Smaller than reference | 97 |
| 10W 10s | 39 | Smaller than reference | 100 |
| 10W 30s | 38 | Smaller than reference | 94 |
| 10W 60s | 23 | Equal to or larger than reference (Measurement was difficult because spots became close to each other) | 76 |
| 10W 90s | 20 | Equal to or larger than reference (Measurement was difficult because spots became close to each other) | 70 |

In Table 9, "○" indicates that the coating agent was dissolved in a volatile polar solvent (mixed solution of water and ethanol), and "×" indicates that the coating agent was not dissolved in the volatile polar solvent. In a case where the amount of the azidophenyl group was not less than 50 mol%, the coating agent was not dissolved in the volatile polar solvent, and it was not possible to coat the substrate with the coating agent.

From Table 10, it was found that the contact angle of the surface of the substrate before the surface of the substrate was coated with the coating agent also affected emission intensity. The contact angle of the surface of the substrate before the surface of the substrate was coated with the coating agent falls within in a range of preferably not less than 20° and less than 80°, and particularly preferably not less than 38° and not more than 51°.

### [Example 4]

In the same manner as in Example 1, an interaction between a protein and a labeled antibody was detected. Instead of the glow discharge, a plasma treatment was carried out (with use of a plasma treatment device Plasma Cleaner PC-300, manufactured by Samco, with 10 W for 10 seconds). As a coating agent, polyvinyl type Az-PEG-1 (substance immobilizing agent (A)) was used, and the concentration thereof was 1.0 wt/vol%. Note, however, that ethylene glycol was added, as a thickener, to a spot solution to be micro-spotted with use of a pin arrayer. As a result, as shown in Table 11, it was possible to stabilize the size of droplets (the size of microspots), and possible to decrease a CV value because the spots did not become close to each other and accordingly measurement did not become difficult to carry out.

**[Table 11]**

| Concentration of ethylene glycol (%) | CV |
|---|---|
| 0 | 9 |
| 50 | 3 |

### Industrial Applicability

The present invention can be used, for example, to analyze and measure a clinical specimen, and can be used for life science studies, medical applications, and the like.

## Claims

1. A substrate that has a surface to which a substance is to be immobilized and on which a layer of a substance immobilizing agent is formed, wherein
the substance immobilizing agent has photoreactive groups and contains a water-soluble polymer or a water-soluble monomer which turns into the water-soluble polymer by polymerization, and
a thickness of the layer of the substance immobilizing agent is not less than 3 nm and less than 500 nm.

2. The substrate as set forth in claim 1, wherein the substance immobilizing agent is one of the following (A), (B), (C), and (D):
(A) the water-soluble polymer is constituted by a polymer which has, in one molecule thereof, at least two photoreactive groups and of which molecule is electrically neutral or the water-soluble polymer has a main chain which contains a polyoxyethylene structure or a polyoxyalkylene structure;
(B) the water-soluble polymer is a water-soluble synthetic polymer or a water-soluble naturally occurring polymer derivative each of which has, in one molecule thereof, at least two photoreactive groups;
(C) the substance immobilizing agent contains (i) a photocrosslinking agent which has, in one molecule thereof, at least two photoreactive groups and (ii) the water-soluble polymer of which molecule is electrically neutral; and
(D) the substance immobilizing agent contains (i) the water-soluble monomer of which molecule is electrically neutral and which has a polymerizable group, (ii) a photopolymerization initiator, and (iii) a photocrosslinking agent which has, in one molecule thereof, at least two photoreactive groups.

3. The substrate as set forth in claim 1 or 2, to which the substance is immobilized via the layer of the substance immobilizing agent.

4. The substrate as set forth in claim 3, to which the substance is immobilized.

5. The substrate as set forth in any one of claims 1 to 4, wherein the substance is selected from the group consisting of polypeptides, enzymes, polysaccharides, nucleic acids, lipids, cells, and components of the cells.

6. The substrate as set forth in any one of claims 2 to 5, wherein the substance immobilizing agent is one of the following (b), (c), and (d):
(b) the water-soluble polymer is constituted by a nonionic water-soluble polymer which has, in one molecule thereof, at least two photoreactive groups, and the nonionic water-soluble polymer is polyalkylene glycol or a polyvinyl-based polymer;
(c) the substance immobilizing agent contains (i) a photocrosslinking agent which has, in one molecule thereof, at least two photoreactive groups and (ii) the water-soluble polymer of which molecule is electrically neutral; and
(d) the substance immobilizing agent contains (i) polyethylene glycol (meth)acrylate as the water-soluble monomer, (ii) a photopolymerization initiator, and (iii) a photocrosslinking agent which has, in one molecule thereof, at least two azide groups as the photoreactive groups.

7. The substrate as set forth in any one of claims 1 to 6, wherein a contact angle with water of the surface of the substrate, to which surface the substance is to be immobilized, is not less than 20° and less than 80°.

8. The substrate as set forth in any one of claims 1 to 7, wherein the photoreactive groups are contained in an amount of not less than 2.5 mol% and less than 50 mol% with respect to the entire water-soluble polymer.

9. A kit for allergy testing or antibody testing, comprising the substrate recited in any one of claims 1 to 8, wherein the substance is an allergen.

10. A kit for biomarker testing by a sandwich method, comprising the substrate recited in any one of claims 1 to 8, wherein the substance is an antibody.

11. A method for testing, with use of the substrate recited in any one of claims 1 to 8 or the kit recited in claim 9 or 10, an interaction between (i) the substance immobilized to the substrate and (ii) a sample.
